(19)

Europäisches
Patentamt
European
Patent Office
Office européen
des brevets

(11)  **EP 1 698 265 B1**

(12)  **EUROPEAN PATENT SPECIFICATION**

(45) Date of publication and mention
of the grant of the patent:
**01.07.2015 Bulletin 2015/27**

(51) Int Cl.:
*A61B 1/04* *(2006.01)*      *A61B 1/00* *(2006.01)*
*A61B 5/06* *(2006.01)*      *A61B 5/07* *(2006.01)*

(21) Application number: **04788430.9**

(22) Date of filing: **30.09.2004**

(86) International application number:
**PCT/JP2004/014402**

(87) International publication number:
**WO 2005/065522 (21.07.2005 Gazette 2005/29)**

(54) **SYSTEM FOR SENSING POSITION IN SUBJECT**

SYSTEM ZUM NACHWEIS DER POSITION IN EINER PERSON

SYSTEME DESTINE A DETECTER UNE POSITION DANS LE CORPS D'UN SUJET

(84) Designated Contracting States:
**DE FR GB**

(30) Priority: **26.12.2003 JP 2003435556**

(43) Date of publication of application:
**06.09.2006 Bulletin 2006/36**

(73) Proprietor: **Olympus Corporation**
**Tokyo 151-0072 (JP)**

(72) Inventors:
• **MINAI, Tetsuo,**
**Hachioji-shi, Tokyo 1920023 (JP)**
• **SHIMIZU, Hatsuo**
**1920024 (JP)**

(74) Representative: **von Hellfeld, Axel**
**Wuesthoff & Wuesthoff**
**Patentanwälte PartG mbB**
**Schweigerstrasse 2**
**81541 München (DE)**

(56) References cited:
**EP-A- 0 667 115**      **WO-A-00/27281**
**WO-A-03/021529**      **JP-A- 4 008 341**
**JP-A- 9 028 662**      **JP-A- 10 230 016**
**JP-A- 2001 046 357**      **JP-A- 2001 179 700**
**JP-A- 2003 117 004**      **US-A- 5 902 238**
**US-A1- 2001 034 475**      **US-A1- 2002 198 439**
**US-A1- 2003 114 742**      **US-A1- 2003 130 792**

**Description**

TECHNICAL FIELD

The present invention relates to a system for detecting a position in a subject, having a body-insertable device, which is introduced into a subject and travels in the subject, and a position transducer that is disposed on the outside of the subject and obtains information of the position of the body-insertable device in the subject.

BACKGROUND ART

In recent years, in the field of endoscopes, a swallowable capsule endoscope has been proposed. The capsule endoscope has an image capturing function and a radio communication function. The capsule endoscope has the function of traveling in the body cavity, for example, in the organs such as the stomach and the small intestine with peristalsis of the organs and sequentially capturing images for a period of time since the capsule endoscope is swallowed from the mouth of a subject for inspection (examination) until it is naturally excreted.

Image data captured in the body by the capsule endoscope as the capsule endoscope travels in the body cavity is sequentially transmitted by radio communication to the outside and stored into a memory provided on the outside. The subject can freely move throughout the period after he/she swallows the capsule endoscope until the capsule endoscope is excreted by carrying a receiver having a radio communication function and a storing function. After the capsule endoscope is excreted, a doctor or nurse can display the images of the organs on a display based on the image data stored in the memory and make a check.

A capsule endoscope has been proposed in which the receiver has the function of detecting the position in the subject of the capsule endoscope to capture, for example, an endoscope image of a specific organ in the subject. As an example of a capsule endoscope system having the position detecting function, a capsule endoscope system using the radio communication function provided in the capsule endoscope is known. That is, the system has a configuration that a receiver provided on the outside of a subject has a plurality of antenna elements, and has the function of receiving a radio signal transmitted from the capsule endoscope by the plurality of antenna elements and, based on intensities received by the antenna elements, detecting the position of the capsule endoscope in the subject (see Patent Document 1, for example).

Patent Document 1: Japanese Patent Application Laid-open No. 2003-19111

DISCLOSURE OF INVENTION

PROBLEM TO BE SOLVED BY THE INVENTION

The conventional capsule endoscope system has, however, a problem such that the precision of detecting the position of the capsule endoscope in the subject is low. The problem is explained in detail hereinbelow.

The capsule endoscope system of the prior art detects the position in the subject, of the capsule endoscope based on a reception intensity distribution in the plurality of antenna elements of the receiver. Such a position detecting mechanism works on condition that, as also described in the paragraph 0018 of Japanese Patent Application Laid-open No. 2003-19111, attenuation of the intensity of a radio signal transmitted from the capsule endoscope is unconditionally determined in accordance with the distance from the capsule endoscope.

However, in reality, values of dielectric constant, conductivity, and the like of structures such as organs existing between the capsule endoscope and the antenna element are different from each other, so that attenuation factors of the radio signal intensities largely vary according to the kinds of the structures and the like. For example, when the liver, blood vessels, and the like exist between the capsule endoscope and the antenna element, a large amount of radio signals is absorbed by the organs and the like. Consequently, as compared with the case where the organs and the like do not exist, the attenuation factor of the radio signal intensity becomes larger and it disturbs accurate position detection.

The present invention has been achieved in view of the above and its object is to realize a system for detecting a position in a subject, capable of accurately detecting the position of a body-insertable device irrespective of the existence of organs and the like in a state where the body-insertable device such as a capsule endoscope is introduced in the subject.

Document WO027281 discloses a system comprising a capsule endoscope having a magnet and a plurality of magnetic sensors disposed on a subject, wherein the plurality of magnetic sensors detect a position and orientation of the capsule endoscope based on the detected magnetic fields. Initial calibration is intended to remove the influence of the earth's magnetic field on the detected values.

Document US2003/0130792 discloses a portable GPS device having azimuth sensors for sensing magnetic fields, the sensed magnetic fields used by a controller to determine whether external magnetic fields are present on the basis of the magnetic charges detected by the azimuth sensors. When it is determined that external magnetic fields are present, the azimuth is not determined. A geomagnetism table can be used such that only one azimuth sensor is needed to determine an azimuth by using a latitude and longitude measurement of the GPS sensor to retrieve stored geomagnetic intensities of the detected position from the geomagnetism table.

MEANS FOR SOLVING PROBLEM

A system for detecting a position in a subject according to claim 1 is provided. The system may comprise a body-insertable device that is introduced into the subject and travels in the subject, and a position transducer that is disposed on the outside of the subject and obtains information of a position of the body-insertable device in the subject, wherein the body-insertable device has a magnetic field generator that outputs a constant magnetic field to the outside of the subject, and the position transducer includes a magnetic field detector that senses magnetic fields; a magnetic field extractor that eliminates a noise magnetic field component from the magnetic fields sensed by the magnetic field detector and extracts a constant magnetic field output from the magnetic field generator; and a position information processor that derives information of the position of the body-insertable device in the subject based on the intensity of the constant magnetic field extracted by the magnetic field extractor.

According to the invention, a body-insertable device has a magnetic field generator that generates a constant magnetic field, and a travel state detecting apparatus detects the position of the body-insertable device based on the intensity of the constant magnetic field generated by the magnetic field generator. The constant magnetic field generated by the magnetic field generator has a characteristic that it attenuates uniformly with distance from the magnetic field generator irrespective of variations in the dielectric constant, magnetic permeability, and the like of structures in the subject 1, so that accurate position detection can be performed and an accurate travel state based on the accurate position information obtained can be derived. Since a magnetic field extractor for extracting a constant magnetic field output from the magnetic field generator by eliminating a noise magnetic field component from a magnetic field sensed by a magnetic field detector is provided, position detection can be performed based on the constant magnetic field obtained by eliminating the noise magnetic field component such as an earth magnetic component. Thus, more accurate position detection can be performed.

EFFECT OF THE INVENTION

A system for detecting a position in a subject according to the invention has a configuration such that a body-insertable device has a magnetic field generator that generates a constant magnetic field, and a travel state detecting apparatus detects the position of the body-insertable device based on the intensity of the constant magnetic field generated by the magnetic field generator. The constant magnetic field generated by the magnetic field generator has a characteristic that it attenuates uniformly with distance from the magnetic field generator irrespective of variations in the dielectric constant, magnetic permeability, and the like of structures in the subject 1, so that accurate position detection can be performed and an accurate travel state based on the accurate position information obtained can be derived. Since a magnetic field extractor for extracting a constant magnetic field output from the magnetic field generator by eliminating a noise magnetic field component from a magnetic field sensed by a magnetic field detector is provided, position detection can be performed based on the constant magnetic field obtained by eliminating the noise magnetic field component such as an earth magnetic component. Thus, more accurate position detection can be performed.

BRIEF DESCRIPTION OF DRAWINGS

Fig. 1 is a schematic view that depicts a general configuration of a system for detecting a position in a subject according to a first embodiment;
Fig. 2 is a schematic view that depicts the configuration of a test capsule as a component of the system for detecting a position in a subject according to the first embodiment;
Fig. 3 is a schematic view that depicts the configuration of a position information processor as a component of the system for detecting a position in a subject according to the first embodiment;
Fig. 4 is a flowchart that explains operations of the position information processor;
Fig. 5 is a schematic view that depicts a mode of deriving the position of a test capsule by the position information processor;
Fig. 6 is a schematic view that depicts the configuration of a test capsule in a modification of the first embodiment;
Fig. 7 is a schematic view that depicts a general configuration of a system for detecting a position in a subject according to a second embodiment;
Fig. 8 is a schematic view that depicts the configuration of a capsule endoscope as a component of the system for detecting a position in a subject according to the second embodiment;

Fig. 9 is a schematic view that depicts the configuration of a position information processor as a component of the system for detecting a position in a subject according to the second embodiment;

Fig. 10 is a flowchart that explains operations of the position information processor; and

Fig. 11 is a schematic view that depicts a mode of deriving the orientation of the capsule endoscope by the position information processor.

EXPLANATIONS OF LETTERS OR NUMERALS

| 1 | subject |
|---|---|
| 2 | test capsule |
| 3 | position transducer |
| 4 | display |
| 5 | portable recording medium |
| 6a to 6h | magnetic field detector |
| 7 | inclination sensor |
| 8 | horizontal in-plane azimuth sensor |
| 9a, 9b | fixing members |
| 10 | position information processor |
| 11 | casing |
| 12 | permanent magnet |
| 13 | filling member |
| 14 | noise magnetic field component database |
| 15 | magnetic field extractor |
| 16 | reference device selector |
| 17 | selector |
| 18 | distance calculator |
| 19 | position calculator |
| 20 | storage unit |
| 21 | case member |
| 22 | liquid |
| 23 | spherical body |
| 24 | weighting member |
| 25 | capsule endoscope |
| 26 | position transducer |
| 27 | position information processor |
| 28 | LED |
| 29 | LED driving circuit |
| 30 | CCD |
| 31 | CCD driving circuit |
| 32 | function executing unit |
| 33 | RF transmitting unit |
| 34 | transmitting antenna unit |
| 35 | system control circuit |
| 36 | receiving antenna unit |
| 37 | separating circuit |
| 38 | power reproducing circuit |
| 39 | booster circuit |
| 40 | capacitor |
| 41 | control information detector |
| 44 | orientation direction database |
| 45 | orientation direction detector |
| 46 | antenna selector |
| 47 | RF receiving unit |
| 48 | image processing unit |
| 49 | storage unit |

| 50 | oscillator |
| 51 | control information input unit |
| 52 | multiplexing circuit |
| 53 | amplifier circuit |
| 54 | power supply unit |
| A1 to An | receiving antennas |
| B1 to Bm | power supply antennas |

BEST MODE(S) FOR CARRYING OUT THE INVENTION

A system for detecting a position in a subject as a best mode for carrying out the invention is explained hereinbelow. It should be noted that the drawings are schematic ones and the relation between thickness and width of each part, the thickness ratio of the parts, and the like are different from real ones. Obviously, the drawings include parts having different relations of dimensions and ratios.

First Embodiment

First, a system for detecting a position in a subject according to the first embodiment is explained. The system for detecting a position in a subject according to the first embodiment has: a test capsule 2 that is introduced to the inside of a subject 1 and functions as an example of a body-insertable device; a position transducer 3 that detects the position in the subject 1 of the test capsule 2; a display 4 displaying position information of the test capsule 2 detected by the position transducer 3; and a portable recording medium 5 for transmitting/receiving information between the position transducer 3 and the display 4.

The display 4 is used for displaying position information of the test capsule 2 obtained by the position transducer 3 and has a configuration like a workstation or the like that displays an image based on data obtained from the portable recording medium 5. Specifically, the display 4 may be constructed to directly display an image by a CRT display, a liquid crystal display, or the like or to output an image to another medium like a printer or the like.

The portable recording medium 5 can be inserted/removed to/from a position information processor 10 that is explained later and the display 4, and has a structure capable of outputting and recording information when inserted to the position information processor 10 and the display 4. Specifically, the portable recording medium 5 is inserted in the position information processor 10 to record information on the position of the test capsule 2 while the test capsule 2 travels in the body cavity of the subject 1. After the test capsule 2 is excreted from the subject 1, the portable recording medium 5 is removed from the position information processor 10 and inserted into the display 4, and the recorded data is read by the display 4. By transmitting data between the position information processor 10 and the display 4 by the portable recording medium 5 such as a compact flash (registered trademark) memory, different from the case where the position information processor 10 and the display 4 are connected to each other by wire, even when the test capsule 2 is traveling in the subject 1, the subject 1 can move freely.

The test capsule 2 is used at the time of conducting a preliminary inspection to check whether or not a narrow part in which passage of a capsule endoscope is difficult exists in the subject 1 prior to introduction of the capsule endoscope or the like into the subject 1. The system for detecting a position in a subject according to the first embodiment is used to check how the test capsule 2 travels in the subject 1. To achieve the purpose, a high-precision position detecting mechanism is provided.

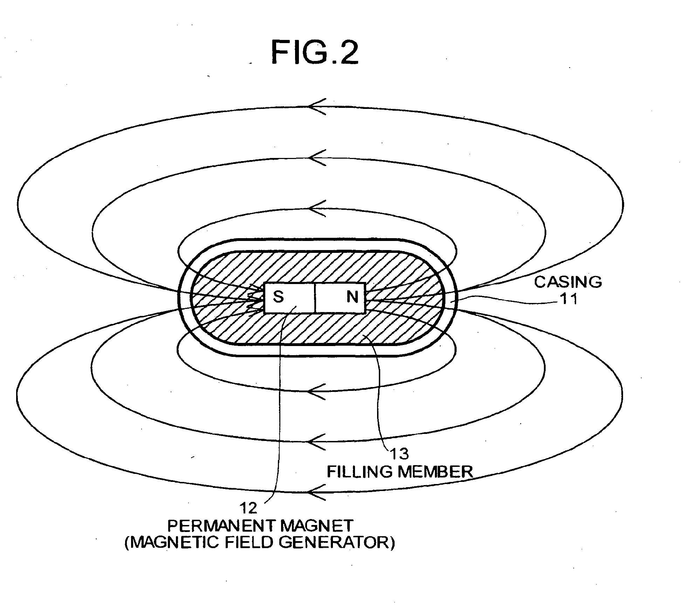

Fig. 2 is a schematic diagram showing the structure of the test capsule 2. As shown in Fig. 2, the test capsule 2 includes: a casing 11 having a capsule shape that is similar to that of a casing of a capsule endoscope; a permanent magnet 12 disposed in the casing 11; and a filling member 13 functioning as a member filling the clearance between the inner surface of the casing 11 and the permanent magnet 12.

The casing 11 is made of, for example, a biocompatible material and has a characteristic such that when the casing 11 remains in the subject 1 for a few days, the material dissolves. By forming the casing 11 of a biocompatible material, there is an advantage such that even if the test capsule 2 introduced in the subject 1 is not excreted to the outside of the subject 1, it is unnecessary to perform an abdominal operation or the like on the subject 1.

The permanent magnet 12 functions as a magnetic field generator in the claims, has a size that can be housed in the casing 11, and is to output a constant magnetic field whose intensity fluctuation with time is ignorable. In place of the permanent magnet 12, for example, a coil that receives constant current and generates a constant magnetic field may be used as the constant magnetic field generator. In the case of using the permanent magnet 12, there is an advantage such that drive power is unnecessary. Thus, it is preferable to construct the magnetic field generator by using the permanent magnet 12.

As shown in Fig. 2, the constant magnetic field generated from the permanent magnet 12 is expressed by a line of magnetic force of a closed curve that is output from the N pole side, travels on the outside of the permanent magnet 12, and enters again on the S pole side. As shown in Fig. 2, the travel direction of the line of magnetic force has location dependency but it can be regarded that the intensity of the constant magnetic field expressed by the line of magnetic force is determined only in accordance with the distance from the test capsule 2. That is, the size of the permanent magnet 12 provided in the test capsule 2 is small enough to be ignored as compared with the distance between the test capsule 2 and magnetic field detectors 6a to 6h. Consequently, magnetic field intensity P at a point apart from the test capsule 2 only by distance "r" is expressed as follows by using a proportional factor $\alpha$.

$$P = \alpha/r^3 \qquad\qquad (1)$$

The system for detecting a position in a subject according to the first embodiment detects the position of the test capsule 2 based on the relation shown in Equation (1) as described later.

The filling member 13 is provided to fill the clearance between the inner face of the casing 11 and the permanent magnet 12 to fix the position of the permanent magnet 12. The material of the filling member 13 does not exert an adverse influence on the subject 1. For example, the filling member 13 is made of barium sulfate. Since barium sulfate can be used as a contrast medium in an X-ray inspection, position detection by an X-ray inspection can be performed in addition to the position detection of the first embodiment. By comparing the results of both of the inspections, more accurate position detection can be performed. Obviously, it is not essential to use barium sulfate as the filling member 13 in the first embodiment and an arbitrary material can be used as long as the material functions as the filling member.

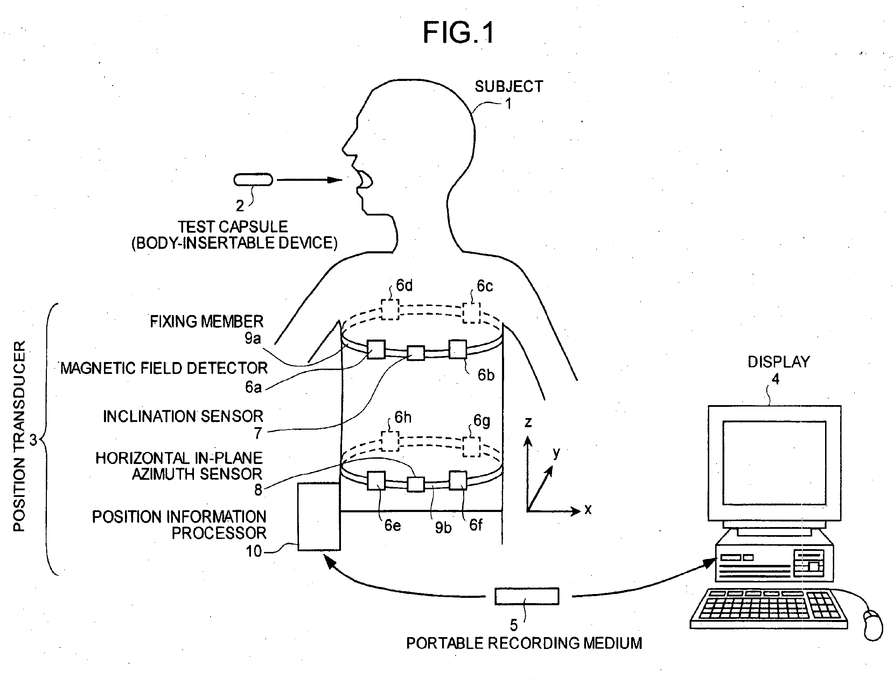

The position transducer 3 is explained. The position transducer 3 detects the position of the test capsule 2 in the subject 1 based on the constant magnetic field output from the test capsule 2. Specifically, the position transducer 3 has, as shown in Fig. 1, the magnetic field detectors 6a to 6h for detecting the intensity of the constant magnetic field output from the test capsule 2, an inclination sensor 7 for sensing the degree of inclination of the subject 1, a horizontal in-plane azimuth sensor 8 for sensing orientation of the subject 1 in a horizontal plane, a fixing member 9a for fixing the magnetic field detectors 6a to 6d and the inclination sensor 7 to the subject 1, a fixing member 9b for fixing the magnetic field detectors 6e to 6h and the horizontal in-plane azimuth sensor 8 to the subject 1, and the position information processor 10 for deriving the position of the test capsule 2 based on the magnetic field intensities detected by the magnetic field detectors 6a to 6h.

Each of the magnetic field detectors 6a to 6h is to detect the direction and intensity of a detected magnetic field in the position where it is disposed. Specifically, each of the magnetic field detectors 6a to 6h is formed by using, for example, an MI (Magneto Impedance) sensor. The MI sensor has a configuration using, for example, an FeCoSiB amorphous wire as a magneto-sensitive medium and senses the magnetic field intensity by using an MI effect that the magnetic impedance of the magneto-sensitive medium largely changes according to an external magnetic field when high-frequency current is passed to the magneto-sensitive medium. Although other magnetic field detectors can be used as the magnetic field detectors 6a to 6h, in the case of using the MI sensor, there is an advantage such that the magnetic field can be detected with particularly high sensitivity.

The magnetic field detectors 6a to 6h are disposed in positions at vertexes of a tube in the first embodiment, and an xyz coordinate system is determined as shown in Fig. 1 in correspondence with the sides of the cube. That is, when the magnetic field detector 6e is set as the origin, the direction from the magnetic field detector 6e toward the magnetic field detector 6f is set as the x axis, the direction from the magnetic field detector 6e toward the magnetic field detector 6h is set as the y axis, and the direction from the magnetic field detector 6e toward the magnetic field detector 6a is set as the z axis. It is assumed that the magnetic field detectors 6a to 6h output magnetic field components parallel to the x axis, y axis, and z axis to a magnetic field extractor 15. As described later, the absolute directions of the xyz coordinate system change according to a change in the posture of the subject 1 or the like. Consequently, a fixed absolute coordinate system is defined as an x'y'z' coordinate system and description will be given so as to distinguish the xyz coordinate system and the x'y'z' coordinate system from each other as necessary. In the x'y'z' coordinate system, the direction of the z' axis is set to the vertical direction.

The inclination sensor 7 is used to sense the inclination degree of the subject 1 and functions as a mode of a vertical direction sensor in the claims. Specifically, the inclination sensor 7 has the function of detecting the angle formed between a predetermined reference direction and the vertical direction. The inclination sensor 7 is disposed in a state where it is fixed to the subject 1 by the fixing member 9a and the positional relations with the magnetic field detectors 6a to 6h similarly fixed to the subject 1 are fixed. Therefore, the inclination sensor 7 has the function of sensing the degree of inclination from the vertical direction in the xyz coordinate system constructed by the magnetic field detectors 6a to 6h. The inclination sensor 7 may sense inclination only in one axis direction and, more preferably, senses inclination in the directions of two axes.

The horizontal in-plane azimuth sensor 8 is used to sense the orientation of the subject 1 in a horizontal plane. Specifically, the horizontal in-plane azimuth sensor 8 is formed by a measuring device such as a gyro and has the function of sensing the angle formed between a predetermined direction and the orientation of the subject 1. Since the horizontal in-plane azimuth sensor 8 is fixed to the subject 1 via the fixing member 9b, the azimuth angle sensed by the horizontal in-plane azimuth sensor 8 has the function of sensing an azimuth fluctuation in the horizontal direction of the xyz coordinate system constructed by the magnetic field detectors 6a to 6h.

The fixing members 9a and 9b are used to fix the magnetic field detectors 6a to 6h, inclination sensor 7, and horizontal in-plane azimuth sensor 8 to the subject 1. Specifically, each of the fixing members 9a and 9b is formed of, for example, an elastic member in an annular shape and is fixed in a state where it is closely attached to the trunk of the subject 1. The magnetic field detectors 6a to 6d and the inclination sensor 7 are fixed to a predetermined position in the subject 1 by the fixing member 9a. The magnetic field detectors 6e to 6h and the horizontal in-plane azimuth sensor 8 are fixed to a predetermined position in the subject 1 by the fixing member 9b. Therefore, by closely attaching the fixing members 9a and 9b to the trunk of the subject 1, the magnetic field detectors 6a to 6h, inclination sensor 7, and horizontal in-plane azimuth sensor 8 are disposed in a state where their relative positions to the subject 1 are fixed.

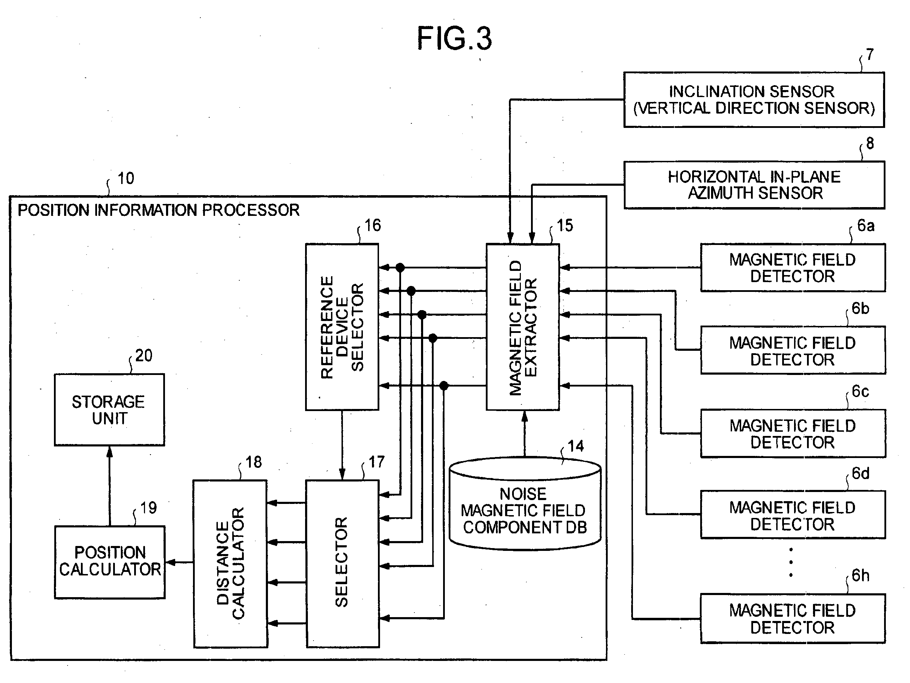

The position information processor 10 is explained. The position information processor 10 extracts intensity of the constant magnetic field output from the test capsule 2 from the magnetic field intensities sensed by the magnetic field detectors 6a to 6h and, based on the extracted magnetic field intensities, derives the position of the test capsule 2. Fig. 3 is a block diagram showing the configuration of the position information processor 10. As shown in Fig. 3, the position information processor 10 has: the magnetic field extractor 15 for extracting the constant magnetic field output from the test capsule 2 from the sensed magnetic fields in the magnetic field detectors 6a to 6h; and a noise magnetic field component database 14 for pre-storing data used at the time of magnetic field extraction by the magnetic field extractor 15. The position information processor 10 also has: a reference device selector 16 that selects a reference magnetic field detector (hereinbelow, called "reference device") from the magnetic field detectors 6a to 6h; a selector 17 that outputs the magnetic field intensity obtained by the predetermined number of magnetic field detectors based on the result of selection by the reference device selector 16; a distance calculator 18 that derives distance between the test capsule 2 and the reference device or the like based on the magnetic field intensity output from the selector 17; a position calculator 19 for deriving the position of the test capsule 2 by performing a computing process using the derived distance and position coordinates of the reference device or the like used for deriving the distance; and a storage unit 20 for storing information of the position of the test capsule 2 obtained by the position calculator 19 into the portable recording medium 5.

The noise magnetic field component database 14 stores noise magnetic fields such as earth magnetic components and the like existing in the region to which the test capsule 2 is introduced in the subject 1. Specifically, the noise magnetic field component database 14 has the function of storing the intensity of the noise magnetic field and the travel direction in the x'y'z' coordinate system and, as necessary, has the function of outputting data to the magnetic field extractor 15.

The magnetic field extractor 15 is provided to eliminate the noise magnetic field component included in the magnetic fields sensed by the magnetic field detectors 6a to 6h and to extract the constant magnetic field output from the permanent magnet 12 in the test capsule 2. The magnetic field extractor 15 has a configuration of receiving not only detection results of the magnetic field detectors 6a to 6h but also the information stored in the noise magnetic field component database 14 and detection results of the inclination sensor 7 and the horizontal in-plane azimuth sensor 8, and has the function of performing magnetic field extraction based on the input information. The detection results of the inclination sensor 7 and the horizontal in-plane azimuth sensor 8 are used for the following reasons.

The travel direction of the noise magnetic field component stored in the noise magnetic field component database 14 is stored based on the x'y'z' coordinate system. On the other hand, a magnetic field sensed by the magnetic field detectors 6a to 6h is sensed based on the xyz coordinate system. Consequently, to eliminate the noise magnetic field component, the information stored in the noise magnetic field component database 14 has to be converted to information in the xyz coordinate system.

The magnetic field extractor 15 therefore derives the relation between the xyz coordinate system and the x'y'z' coordinate system at the time of detecting a magnetic field based on the results of detection by the inclination sensor 7 and the horizontal in-plane azimuth sensor 8 and, based on the derived relation between the coordinate systems, performs a process of transforming the coordinates of data of the noise magnetic field component. After the coordinate transforming process, the magnetic field extractor 15 derives a difference value between the magnetic fields sensed by the magnetic field detectors 6a to 6h with respect to the x, y, and z directions and the noise magnetic field components, and outputs the derived difference value as the constant magnetic field output from the permanent magnet 12 to the reference device selector 16 and the selector 17.

The reference device selector 16 has the function of selecting the largest value of the detected magnetic field intensity from the magnetic field detectors 6a to 6h. Specifically, the reference device selector 16 compares the magnetic field intensity values output from the magnetic field detectors 6a to 6h with each other, selects the magnetic field detector (reference device) that has output the largest magnetic field intensity value, and outputs information specifying the reference device (for example, information indicating the reference device among the magnetic field detectors 6a to 6h) to the selector 17.

The selector 17 selects a plurality of magnetic field detectors based on the result of selection of the reference device selector 16 and outputs the magnetic field intensities obtained by the magnetic field detectors selected by itself (selected devices) and the magnetic field intensity obtained by the reference device to the distance calculator 18. Specifically, the selector 17 has the function of selecting three magnetic field detectors disposed in directions orthogonal to each other with respect to the reference device. That is, in the system for detecting a position in a subject according to the first embodiment, as also shown in Fig. 1, the magnetic field detectors 6a to 6h are disposed so as to form vertexes of a cube, so that three magnetic field detectors positioned in directions orthogonal to each other always exist for an arbitrary magnetic field detector, and the selector 17 has the function of selecting the three magnetic field detectors as selected devices.

The distance calculator 18 derives the distances among the reference device, the selected devices, and the test capsule 2 based on the magnetic field intensities received via the selector 17. Specifically, the distance calculator 18 has the function of deriving the distance between the magnetic field detector that has detected the magnetic field intensity and the test capsule 2 by performing the computing process shown by Equation (1) with respect to the input magnetic field intensity.

The position calculator 19 derives the position of the test capsule 2 by performing a predetermined computing process based on the distance between the magnetic field detector selected as the reference device or the like and the test capsule 2. The position calculator 19 also has the function of outputting the result of derivation to the storage unit 20 after deriving the position of the test capsule 2.

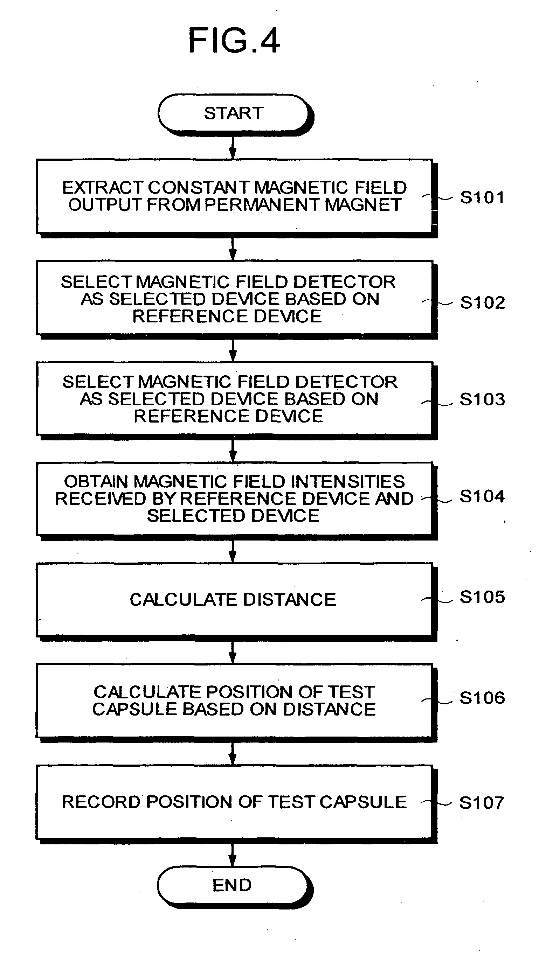

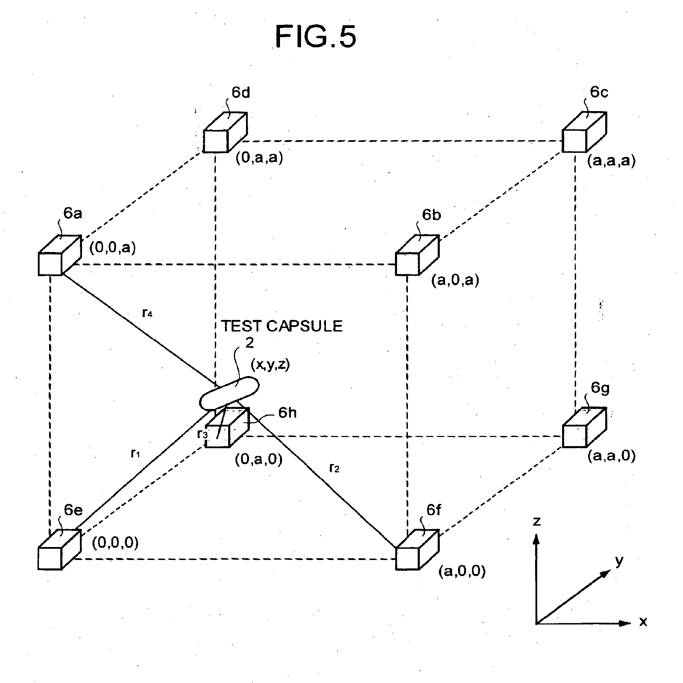

The operation of the position information processor 10 in the first embodiment is explained. Fig. 4 is a flowchart showing the operation of the position information processor 10, and Fig. 5 is a schematic diagram for explaining the algorithm of the position deriving operation. In Fig. 5, the length of one side of a cube constructed by the magnetic field detectors 6a to 6h is set as "a". As described later, the position of the magnetic field detector 6e selected as a reference device is set as the origin, the direction from the magnetic field detector 6e toward the magnetic field detector 6f is set as an x direction, the direction from the magnetic field detector 6e toward the magnetic field detector 6h is set as a y direction, and the direction from the magnetic field detector 6e toward the magnetic field detector 6a is set as a z direction. The positions of the magnetic field detectors 6a to 6h are determined based on the xyz coordinate system, and the position of the test capsule 2 in the xyz coordinate system is expressed as (x, y, z). The operation of the position information processor 10 is explained hereinbelow by properly referring to Figs. 4 and 5.

First, the position information processor 10 extracts the constant magnetic field output from the permanent magnet 12 from the magnetic fields sensed by the magnetic field detectors 6a to 6d by the magnetic field extractor 15 (step S101). Specifically, the magnetic field extractor 15 transforms information in the x'y'z' coordinate system regarding the noise magnetic field component supplied from the noise magnetic field component database 14 to information in the xyz coordinate system based on a detection result obtained by the inclination sensor 7 and the like. The magnetic field extractor 15 derives the difference value between the noise magnetic field component subjected to the coordinate transformation and the magnetic fields sensed by the magnetic field detectors 6a to 6h, thereby extracting the constant magnetic field.

After that, the position information processor 10 selects the magnetic field detector that has sensed the highest intensity of the constant magnetic field extracted by the magnetic field extractor 15 by the reference device selector 16 (step S102). The example of Fig. 5 shows a case where the magnetic field detector 6e is selected as the magnetic field detector sensing the highest intensity. In the following description, it is also assumed that the magnetic field detector 6e is the reference device.

The position information processor 10 selects three devices to be selected by the selector 17 based on the reference device selected in step S102 (step S103), and outputs the intensity of the constant magnetic field sensed by the reference device and the selected devices and extracted by the magnetic field extractor 15 to the distance calculator 18 (step S104). In the example of Fig. 5, the magnetic field detectors 6f, 6h, and 6a are disposed in the directions orthogonal to each other with respect to the magnetic field detector 6e as a reference device, so that the selector 17 selects those magnetic field detectors as selected devices.

After that, the position information processor 10 derives the distance from the test capsule 2 by the distance calculator 18 based on the intensity of the constant magnetic field obtained based on the detection result of the reference device selected in step S102 and the intensities of the constant magnetic fields obtained based on the detection results of the devices selected in step S103 (step S105). Specifically, the distance calculator 18 derives the distance by performing computation of Equation (1) using the magnetic field intensities input via the selector 17. In the example of Fig. 5, the distance calculator 18 derives distances $r_1$, $r_2$, $r_3$, and $r_4$ between the test capsule 2 and the magnetic field detectors 6e, 6f, 6b, and 6a, respectively, based on the intensities of the constant magnetic fields obtained based on the detection results of the reference device and the selected devices.

The position information processor 10 derives the position of the test capsule 2 by the computing process in the position calculator 19 (step S106). Specifically, the position of the test capsule 2 is derived by deriving the x coordinate, y coordinate, and z coordinate of the test capsule 2, so that the coordinates of the test capsule 2 are derived by using the coordinates of the magnetic field detectors 6e, 6f, 6h,and 6a and the values of distances derived in step S105.

For example, the position coordinates (x, y, z) of the test capsule 2 can be geometrically derived from the positional relations shown in Fig. 5 and, concretely, can be derived by solving the following equations.

$$(x-0)^2 + (y-0)^2 + (z-0)^2 = r_1^2 \qquad (2)$$

$$(x-a)^2 + (y-0)^2 + (z-0)^2 = r_2^2 \qquad (3)$$

$$(x-0)^2 + (y-a)^2 + (z-0)^2 = r_3^2 \qquad (4)$$

$$(x-0)^2 + (y-0)^2 + (z-a)^2 = r_4^2 \qquad (5)$$

In Equations (2) to (5), the number of unknown letters is three so that three equations are theoretically sufficient. At the time of actual position detection, however, to suppress deterioration in precision of the position detection of the test capsule 2 due to a positional deviation of the magnetic field detectors 6a to 6h and the distance derivation error or the like, after solving Equations (2) to (5), the coordinates of the magnetic field detector and the like are corrected so that the values x, y, and z are unconditionally determined.

Finally, the position information processor 10 stores the position of the test capsule 2 derived in step S106 by the storage unit 20 (step S107). Specifically, while the test capsule 2 is introduced in the subject 1, the portable recording medium 5 is inserted in the storage unit 20, so that the storage unit 20 records the position information obtained in step S106 into the portable recording medium 5.

The processes in steps S102 to S107 are repeatedly performed at predetermined time intervals. As a result, the portable recording medium 5 records information of travel in the subject 1 of the test capsule 2. After the test capsule 2 is excreted to the outside of the subject 1, the portable recording medium 5 is inserted to the display 4. The user grasps how the test capsule 2 travels in the subject 1 based on the result of recording displayed on the display 4 and determines the location of a narrow region existing in the subject 1 or the like from the grasped result.

The advantages of the system for detecting a position in a subject according to the first embodiment is explained. First, the system for detecting a position in a subject according to the first embodiment derives the position of the test capsule 2 based on the constant magnetic field output from the permanent magnet 12 in the test capsule 2. Different from electromagnetic waves and the like, the constant magnetic field has a characteristic that its intensity attenuates almost unconditionally irrespective of fluctuations in physical parameters such as dielectric constant, magnetic permeability, and the like in a propagation region, so that the relation of Equation (1) is excellently satisfied. Therefore, the system has an advantage such that position detection can be performed with higher precision even in a space like the inside of a human body where organs and the like whose physical parameters are different from each other exist as compared with the case of position detection using electromagnetic waves or the like.

Another advantage of using the constant magnetic field is that burden on the subject 1 applied when the test capsule 2 is introduced into the subject 1 is lessened. For the reasons, the system for detecting a position in a subject according to the first embodiment has an advantage such that deterioration in precision of the position detection due to variations of environments around the test capsule 2 is suppressed. Consequently, for example, at the time of introducing the test capsule 2 into the subject 1, it is unnecessary to impose limitations such as restriction on eating and drinking like in other inspecting methods. Therefore, the subject 1 can live normal life also at the time of an inspection using the test capsule 2 and the burden on the subject 1 in the inspection can be lessened.

Modification

A modification of the system for detecting a position in a subject according to the first embodiment is explained. In the modification, the arrangement of the permanent magnet 12 is devised so that the constant magnetic field output from the test capsule becomes always in the vertical direction.

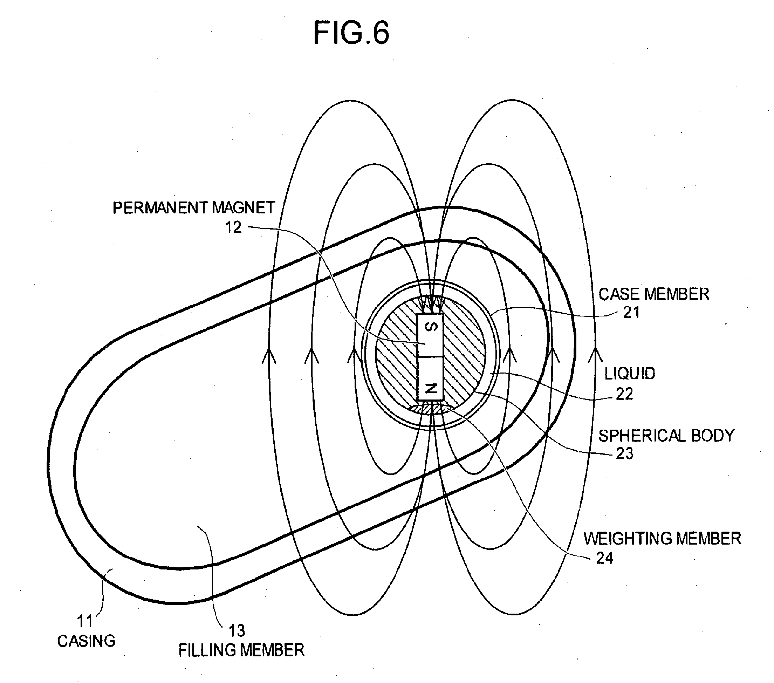

Fig. 6 is a schematic view that depicts the configuration of a test capsule in the modification. As shown in Fig. 6, the test capsule in the modification has the casing 11 and the filling member 13 in a manner similar to the first embodiment and the permanent magnet 12 is housed in a case member 21. Specifically, the permanent magnet 12 is held in a spherical body 23 and the spherical body 23 is disposed in a state where it floats in a liquid 22 held in the case member 21. In the spherical body 23, a weighting member 24 is disposed in the magnetic field output direction from the permanent magnet 12. The spherical body 23 is stabilized in a state where the weighting member 24 is positioned at the bottom in the vertical direction irrespective of fluctuations in the orientation of the test capsule as shown in Fig. 6.

With the configuration, in the modification, the constant magnetic field output from the permanent magnet 12 in the test capsule is always in the vertical direction. On the other hand, the earth magnetic component as a main noise magnetic field component travels almost in the horizontal direction, so that it is sufficient for the magnetic field extractor 15 to extract only the components in the vertical direction from the magnetic fields sensed by the magnetic field detectors 6a to 6h.

To extract the components in the vertical direction from the sensed magnetic fields, the magnetic field extractor 15 extracts only components parallel to the vertical direction sensed by the inclination sensor 7 from the magnetic fields sensed by the magnetic field detectors 6a to 6h, thereby enabling extraction of the constant magnetic field output from the permanent magnet 12 to be completed. Therefore, in the modification, the position transducer 3 does not have to have the horizontal in-plane azimuth sensor 8, and the noise magnetic field component database 14 can be omitted from the position information processor 10. Further, in the modification, the magnetic field extractor 15 does not have to perform a process of deriving a difference value and the like. Thus, by using the test capsule shown in Fig. 6, a system for detecting a position in a subject, in which the influence of noise magnetic field components is reduced can be realized with a simple configuration.

Second Embodiment

Next, a system for detecting a position in a subject according to the second embodiment is explained. The system for detecting a position in a subject according to the second embodiment has: a capsule endoscope, as a device introduced into a subject, including not only the constant magnetic field generator but also a predetermined function executing unit and a radio unit; and a position information processor that detects not only the position of the capsule endoscope in a subject but also the direction of the major axis, that is, orientation of the capsule endoscope having a shape of an ellipsoid of revolution based on the constant magnetic field generated by the constant magnetic field generator and, based on the result of detection, switches a plurality of antennas receiving radio signals transmitted from the capsule endoscope.

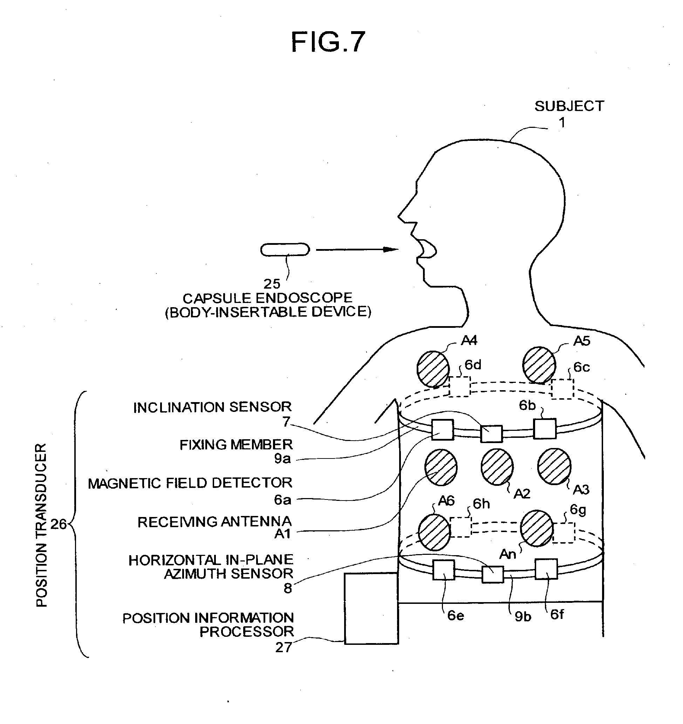

Fig. 7 is a schematic diagram showing a general configuration of the system for detecting a positioning a subject according to the second embodiment. As shown in Fig. 7, the system for detecting a position in a subject according to the second embodiment has a capsule endoscope 25 as an example of the body-insertable device and a position transducer 26. Although elements corresponding to the display 4 and the portable recording medium 5 in the first embodiment are not shown in Fig. 7, it does not mean that those elements are excluded in the second embodiment. In the system for detecting a position in a subject according to the second embodiment, elements having the same reference numerals and names as those of the first embodiment have the same configurations and actions as those of the first embodiment unless otherwise specified in the following.

The position transducer 26 has, as shown in Fig. 7, the magnetic field detectors 6a to 6h, the inclination sensor 7, the horizontal in-plane azimuth sensor 8, the fixing members 9a and 9b for fixing the magnetic field detectors 6a to 6h and the like to the subject 1, receiving antennas A1 to An for receiving radio signals transmitted from the capsule endoscope 25, and a position information processor 27 for processing the information obtained by the magnetic field detectors 6a to 6h and the receiving antennas A1 to An and deriving information of the position in the subject 1, of the capsule endoscope 25. Although not shown in Fig. 7, the position transducer 26 also has power supply antennas B1 to Bm that is explained later.

The receiving antennas A1 to An are to receive radio signals transmitted from the capsule endoscope 25. As described later, the capsule endoscope 25 in the second embodiment has the function of capturing an image of the inside of the subject 1 and transmitting the image to the outside by radio. The receiving antennas A1 to An have the configuration of receiving a radio signal transmitted from the capsule endoscope 25 and outputting it to the position information processor 27. The receiving antennas A1 to An are constructed by, concretely, for example, a loop antenna and fixing means for fixing the loop antenna to the subject 1. When radio signals are transmitted from the capsule endoscope 25, the radio signals may be received by all of the receiving antennas A1 to An. However, in the second embodiment, the radio signal is received by using a receiving antenna that is determined to be most adapted to reception by an antenna selector 46 that is explained later among the plurality of receiving antennas A1 to An.

The capsule endoscope 25 has, like the test capsule 2 in the first embodiment, the permanent magnet 12 as the magnetic field generator. The capsule endoscope 25 also has the function of a function executing unit for executing a predetermined function on the inside of the subject 1 and the function of a receiver for receiving a radio signal transmitted from the position transducer 26, and has components corresponding to the functions.

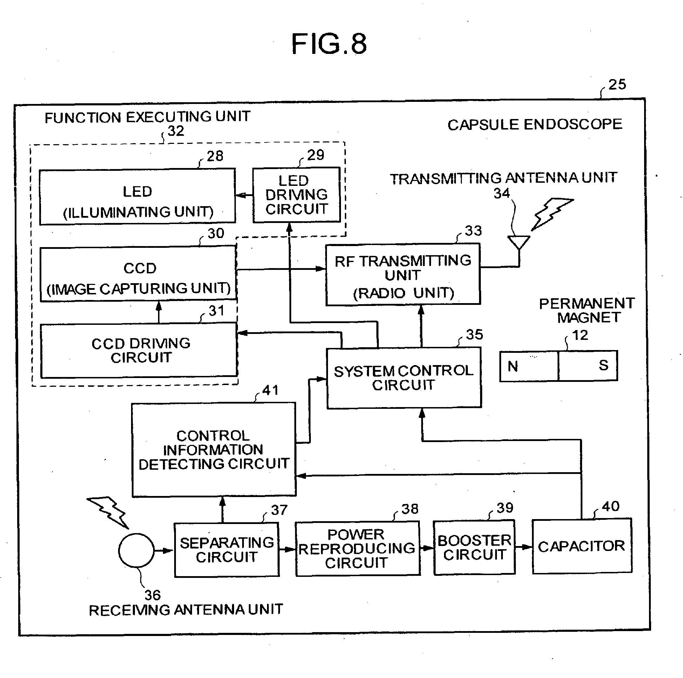

The capsule endoscope 25 has the configuration for realizing the function of the function executing unit for executing a predetermined function and the function of a transmitter for transmitting information obtained by the function executing unit by radio. Fig. 8 is a block diagram showing the configuration of the capsule endoscope 25. Specifically, the capsule endoscope 25 has: an LED 28 functioning as an illuminating unit for illuminating an image capturing region at the time of capturing an image of the inside of the subject 1; an LED driving circuit 29 that controls a driving state of the LED 28; a CCD 30 functioning as an image capturing unit for capturing a reflection light image from the region illuminated by the LED 28; and a CCD driving circuit 31 that controls a driving state of the CCD 30. The LED 28, LED driving circuit 29, CCD 30, and CCD driving circuit 31 are defined as a function executing unit 32 executing a predetermined function as a whole.

The capsule endoscope 25 has: an RF transmitting unit 33 that modulates image data captured by the CCD 30, thereby generating an RF signal; a transmitting antenna unit 34 as a radio unit for transmitting the RF signal output from the RF transmitting unit 33 by radio; and a system control circuit 35 for controlling the operation of the LED driving circuit 29, CCD driving circuit 31, and RF transmitting unit 33.

By having the mechanisms, the capsule endoscope 25 obtains image data of a region to be tested that is illuminated by the LED 28 by the CCD 30 while the capsule endoscope 25 is introduced in the subject 1. The captured image data is converted to an RF signal by the RF transmitting unit 33 and, after that, the RF signal is transmitted to the outside via the transmitting antenna unit 34.

The capsule endoscope 25 also has a configuration for receiving a radio signal transmitted from the position transducer 26. Specifically, the capsule endoscope 25 has: a receiving antenna unit 36 for receiving a radio signal sent from the position transducer 26 side; and a separating circuit 37 for separating the power supply signal from the signal received by the receiving antenna unit 36. The capsule endoscope 25 also has: a power reproducing circuit 38 for reproducing power from the separated power supply signal; a booster circuit 39 for boosting the reproduced power; and a capacitor 40 for storing the boosted power. The capsule endoscope 25 also has a control information detecting circuit 41 for detecting a control information signal from the components separated from the power supply signal by the separating circuit 37 and outputting the detected control information signal to the system control circuit 35. The system control circuit 35 also has the function of distributing drive power supplied from the capacitor 40 to the other elements.

By having the mechanisms, first, the capsule endoscope 25 receives the radio signal sent from the position transducer 26 side by the receiving antenna unit 36 and separates the power supply signal and the control information signal from the received radio signal by the separating circuit 37. The control information signal separated by the separating circuit 37 is output to the system control circuit 35 via the control information detecting circuit 41 and used for driving and controlling the LED 28, CCD 30, and RF transmitting unit 33. On the other hand, the power supply signal is reproduced as power by the power reproducing circuit 38. The potential of the reproduced power is boosted to potential adapted to the capacitor 40 by the booster circuit 39, and the boosted potential is stored in the capacitor 40.

The configuration of the position information processor 27 is explained. The position information processor 27 has not only the function of deriving the position of the capsule endoscope 25 but also the function of deriving the orientation and, further, has the function of a receiver for receiving a radio signal transmitted from the capsule endoscope 25 and the function of a transmitter for transmitting a predetermined signal by radio to the capsule endoscope 25. The configuration of the position information processor 27 is explained hereinbelow with respect to each of the components corresponding to the functions.

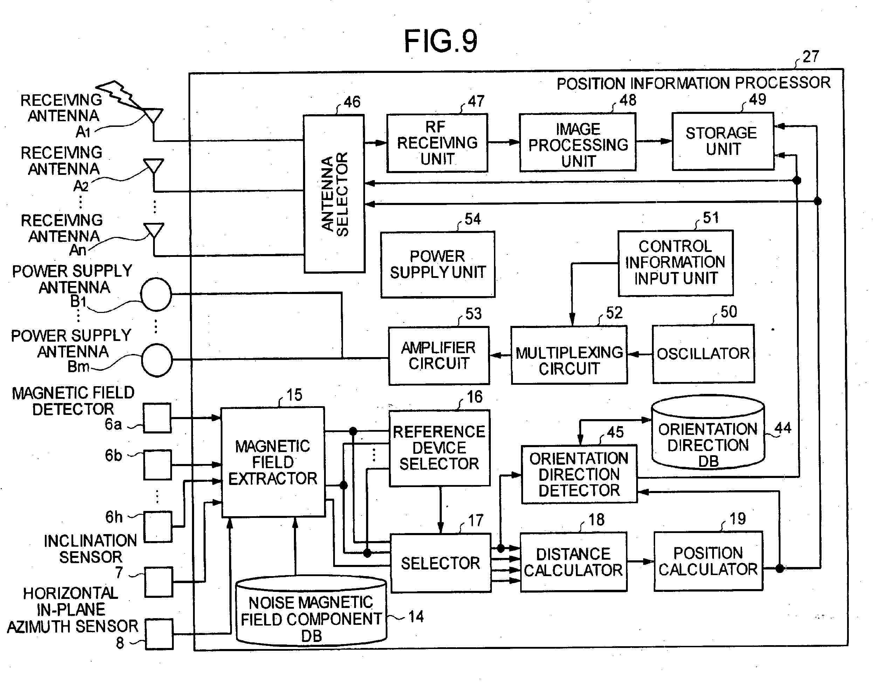

Fig. 9 is a block diagram showing the configuration of the position information driving apparatus 27. The position information processor 27 in the second embodiment has, as elements for detecting the position in the subject 1 of the capsule endoscope 25, the noise magnetic field component database 14, the magnetic field extractor 15, the reference device selector 16, selector 17, distance calculator 18, and position calculator 19. In the second embodiment, the magnetic field detectors 6a to 6h output not only the magnetic field intensity but also the magnetic field direction to the position information processor 27, so that the second embodiment is different from the first embodiment with respect to the following points. The reference device selector 16 extracts the magnetic field intensity from the information output from the magnetic field detectors 6a to 6h to select a reference device, and the distance calculator 18 has the function of deriving distance by extracting the magnetic field intensities received by the reference device and the selected devices from the information received from the selector 17. The operation of detecting the position of the capsule endoscope 25 in the second embodiment is almost the same as that in the first embodiment and its detailed description will not be repeated.

The position information processor 27 also has: an orientation direction database 44 used at the time of detecting the orientation of the capsule endoscope 25 as described later, and an orientation direction detector 45 that detects the orientation of the capsule endoscope 25 based on the magnetic field direction in a predetermined magnetic field detector 6, output from the selector 17. In the orientation direction database 44, the data of the intensity of the magnetic field detected by the magnetic field detector 6 and the orientation of the capsule endoscope 25 with respect to the positional relation between the magnetic field detector 6 and the capsule endoscope 25 is pre-stored. Specific operations of the orientation direction database 44 and the orientation direction detector 45 is explained in detail later.

The position information processor 27 also has the function of a receiver that receives image data of the inside of the subject 1, which is sent from the capsule endoscope 25 by radio. Specifically, the position information processor 27 has: the antenna selector 46 for selecting an antenna used for data reception from the receiving antennas A1 to An; an RF receiving unit 47 that performs a predetermined process such as demodulation on a radio signal received by the selected receiving antenna, extracts image data captured by the capsule endoscope 25 from the radio signal, and outputs the extracted image data; an image processing unit 48 for performing a necessary process on the output image data; and a storage unit 49 for storing the processed image data.

The antenna selector 46 is used to select the receiving antenna that is most adapted to receive the radio signal transmitted from the capsule endoscope 25. Specifically, the antenna selector 46 grasps the positions of the receiving antennas A1 to An in advance, and receives information of the position of the capsule endoscope 25 derived by the position calculator 19 and information of the orientation of the capsule endoscope 25 derived by the orientation direction detector 45. The antenna selector 46 has the function of selecting a receiving antenna that is estimated to have the most excellent reception sensitivity in the relation between the position and orientation of the capsule endoscope 25 and outputting a radio signal received by the selected receiving antenna to the RF receiving unit 47.

The storage unit 49 has the function of storing image data output from the image processing unit 48 and the position and orientation of the capsule endoscope 25 at the time point the output image data is captured in a state where the image data and the position and orientation of the capsule endoscope 25 are associated with each other. The position information processor 27 has the configuration of outputting the information obtained by the position calculator 19, orientation direction detector 45, and image processing unit 48 to the storage unit 49 as also shown in Fig. 9, and the storage unit 49 has the function of storing the information in the associated state. As a result, in the storage unit 49, the image data of a predetermined region in the subject 1 and the position and orientation of the capsule endoscope 25 at the time point the image data is captured is stored in a state where the image data and the position and orientation of the capsule endoscope 25 are associated with each other.

The position information processor 27 also has the function of generating a power supply signal and the like to be transmitted to the capsule endoscope 25 and outputting the power supply signal to the power supply antennas B1 to Bm. Specifically, the position information processor 27 has: an oscillator 50 having the function of generating a power supply signal and the function of specifying an oscillation frequency; a control information input unit 55 that generates a control information signal for controlling the drive state of the capsule endoscope 25; a multiplexing circuit 52 for combining the power supply signal and the control information signal; and an amplifier circuit 53 for amplifying the intensity of the combined signal. The signal amplified by the amplifier circuit 53 is sent to the power supply antennas B1 to Bm and transmitted to the capsule endoscope 25. The position information processor 27 has a power supply unit 54 having a predetermined storage, an AC power adapter, or the like, and the elements of the position information processor 27 use the power supplied from the power supply unit 54 as driving energy.

The significance of detecting the orientation of the capsule endoscope 25 and the details of the orientation detecting operation in the system for detecting a position in a subject according to the second embodiment is explained. As mentioned above, the system for detecting a position in a subject according to the second embodiment has a configuration such that the capsule endoscope 25 has a predetermined function executing unit, and information obtained by the function executing unit is transmitted to the position transducer 26 side by radio. Consequently, the position transducer 26 has the plurality of receiving antennas A1 to An for receiving transmitted radio signals and selects a receiving antenna that is the optimum for reception from the plurality of receiving antennas A1 to An by the antenna selector 46.

As an algorithm of selecting the optimum receiving antenna from the plurality of receiving antennas A1 to An, first, the optimum receiving antenna is determined by the positional relation with the capsule endoscope 25. For example, on assumption that the ratio signal transmitted from the capsule endoscope 25 attenuates with distance, the position of the capsule endoscope 25 is derived by using a position detecting mechanism similar to that of the first embodiment and a receiving antenna closest to the derived position is used.

In the case of receiving the radio signal from the capsule endoscope, however, it is not always proper to select the receiving antenna based on only the positional relation with the antenna. Due to the facts such that the transmitting antenna unit 34 used for radio transmission from the capsule endoscope 25 is constructed by, for example, a loop antenna, the transmitting antenna unit 34 does not transmit radio signals having uniform intensity in all of directions but transmits radio signals with some directivity. Therefore, it is preferable that the receiving antenna that is most adapted to receive a radio signal from the capsule endoscope be determined by considering not only the positional relation with the capsule endoscope but also the directivity of the radio signal sent from the transmitting antenna unit 34. Since the transmitting antenna unit 34 is fixed to the inside of the capsule endoscope 25, to detect the orientation of a radio signal transmitted, it is important to grasp the orientation of the capsule endoscope 25 in the subject 1. Based on the circumstances, in the second embodiment, in a manner similar to the first embodiment, the mechanism of detecting the position in the subject 1 of the capsule endoscope 25 is provided and, in addition, the orientation direction database 44 and the orientation direction detector 45 are newly provided, thereby detecting the orientation of the capsule endoscope 25.

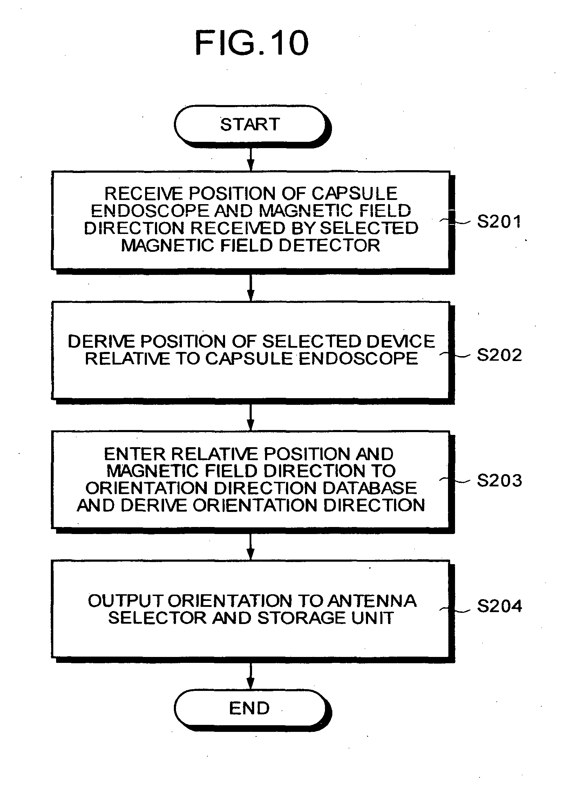

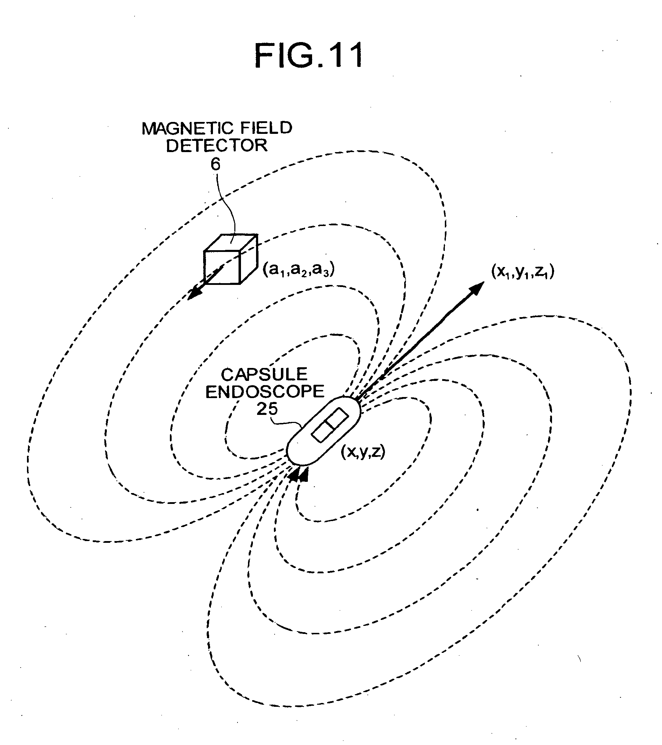

Fig. 10 is a flowchart that explains the operation of detecting the orientation of the capsule endoscope 25 in the orientation direction detector 45 in the second embodiment. Fig. 11 is a schematic view that depicts the relation between the orientation of the capsule endoscope and the magnetic field detector 6. The operation of the orientation direction detector 45 is explained hereinbelow by properly referring to Figs. 10 and 11.

First, the orientation direction detector 45 receives the position of the capsule endoscope 25 and the direction of the constant magnetic field detected by the magnetic field detector selected from the plurality of magnetic field detectors 6a to 6h and extracted by the magnetic field extractor 15 (step S201). Although an algorithm of selecting the magnetic field detector 6 may be arbitrary, in the second embodiment, for example, the magnetic field detector 6 whose detection magnetic field intensity is the highest is selected. In the example of Fig. 11, the coordinates $(a_1, a_2, a_3)$ of the selected magnetic field detector 6 and the magnetic field directions expressed by direction vectors of the arrows are grasped by the orientation direction detector 45.

The orientation direction detector 45 derives the position of the magnetic field detector 6 selected in step S201 relative to the capsule endoscope 25 (step S202). Specifically, the orientation direction detector 45 receives the position of the capsule endoscope 25 derived by the position calculator 19 and derives the coordinates of the magnetic field detector 6 selected in step S201 relative to the capsule endoscope 25. In the example of Fig. 11, based on the coordinates $(a_1, a_2, a_3)$ of the selected magnetic field detector 6 and the coordinates $(x, y, z)$ of the capsule endoscope 25, relative position coordinates $(a_1 - x, a_2 - y, a_3 - z)$ of the magnetic field detector 6 when the position of the capsule endoscope 25 is set as the origin are derived.

After that, the orientation direction detector 45 inputs the magnetic field directions input in step S201 and the relative position of the magnetic field detector 6 selected in step S202 into the orientation direction database 44 and obtains data regarding the orientation of the capsule endoscope 25 (step S203). As shown in Fig. 11, there is the property that the direction of the constant magnetic field output from the permanent magnet 12 provided in the capsule endoscope 25 is unconditionally determined by the orientation of the capsule endoscope 25 and the position relative to the capsule endoscope 25. Consequently, the orientation of the capsule endoscope 25, the coordinates relative to the capsule endoscope 25, and the direction of the constant magnetic field in the relative coordinates are pre-stored in a state where they are associated with each other in the orientation direction database 44. Consequently, by entering the relative coordinates of the magnetic field detector 6 and the directions of the detected constant magnetic field into the orientation direction database 44, the orientation of the capsule endoscope 25 can be extracted. In the example of Fig. 11, based on the output result of the orientation direction database 44, the orientation of the capsule endoscope 25 is derived as $(x_1, y_1, z_1)$.

Finally, the orientation direction detector 45 outputs the obtained data regarding the orientation of the capsule endoscope 25 to the antenna selector 46 and the storage unit 49 (step S204). The antenna selector 46 selects a receiving antenna most suitable for reception based on the data regarding the orientation and the information regarding the position that is output from the position calculator 19, and the storage unit 49 stores the orientation of the capsule endoscope 25 at predetermined time so as to be associated with the image data and the position information of the capsule endoscope 25.

The advantages of the system for detecting a position in a subject according to the second embodiment is explained. First, in the system for detecting a position in a subject according to the second embodiment, in a manner similar to the first embodiment, the capsule endoscope 25 has therein the permanent magnet 12, a constant magnetic field output from the permanent magnet 12 is extracted from a detected magnetic field, and the position of the capsule endoscope 25 is detected based on the detected constant magnetic field. As already mentioned above, the constant magnetic field has a characteristic that it attenuates almost uniformly with distance irrespective of the values such as dielectric constant, magnetic permeability, and the like in organs and the like in the subject 1. Consequently, there is an advantage such that the position of the capsule endoscope 25 can be accurately detected more than the case of performing position detection by using a radio signal.

The system for detecting a position in a subject according to the second embodiment has a configuration that the orientation of the capsule endoscope 25 is detected based on the constant magnetic field output from the permanent magnet 12. Like the case of position detection, the constant magnetic field output from the permanent magnet 12 is not easily influenced by the structures in the subject 1, and has a property such that the magnetic field direction in a predetermined position is almost unconditionally determined based on the orientation of the capsule endoscope 25 and the position relative to the capsule endoscope 25. Therefore, by preliminarily deriving the azimuth distribution of the constant magnetic fields output from the permanent magnet 12, storing it in the orientation direction database 44, and referring to the orientation direction database 44 based on the information obtained by the magnetic field detector 6, the orientation of the capsule endoscope 25 can be accurately detected.

Further, since the system for detecting a position in a subject according to the second embodiment has a configuration that the orientation of the capsule endoscope 25 is detected based on the constant magnetic fields in a manner similar to the case of position detection, there is an advantage such that the system can be realized with a simple configuration. That is, in the system for detecting a position in a subject according to the second embodiment, it is unnecessary to add a new element into the capsule endoscope 25 in order to provide the function of detecting the orientation of the capsule endoscope 25. Thus, a small and low-cost position information detecting system can be constructed.

The system for detecting a position in a subject according to the second embodiment has a configuration that the antenna selector 46 selects a receiving antenna based on the derived position and orientation of the capsule endoscope 25. The reception sensitivity of the radio signal in the receiving antenna depends on the distance from the capsule endoscope 25 and the orientation of the transmitting antenna unit 34 in the capsule endoscope 25. Therefore, a receiving antenna to be used can be accurately selected based on the position and orientation of the capsule endoscope 25, and a position information detecting system capable of receiving a radio signal transmitted from the capsule endoscope 25 always at high sensitivity can be realized.

Further, the system for detecting a position in a subject according to the second embodiment has a configuration of outputting image data of the inside of the subject 1 captured and the derived position and orientation of the capsule endoscope 25 to the storage unit 49. Therefore, image data obtained by the capsule endoscope 25 and the derived position and orientation at the time of image capture of the capsule endoscope 25 can be stored so as to be associated with each other. At the time of displaying image data on the display 4, only the image data positioned in a predetermined range can be designated to be displayed. In other words, every image data is not displayed on the display 4 but image data of a region of interest of the user, for example, image data of only the small intestine can be displayed on the display 4. Thus, the position information detecting system that is convenient to doctors and the like can be realized.

Although the invention is explained above by the first and second embodiments, the invention is not limited to the foregoing embodiments. Various embodiments, modifications, and application examples can be made by those skilled in the art. For example, the system for detecting a position in a subject according to the first embodiment may have a configuration of deriving the orientation of the test capsule 2 in a manner similar to the second embodiment or may employ the configuration of the test capsule in the modification of the first embodiment in place of that of the capsule endoscope 25 in the second embodiment.

In the first and second embodiments, the plurality of magnetic field detectors 6 are disposed on the outer surface of the subject 1 so as to detect the vertexes of a cube. However, the invention is not limited to the layout. With respect to the magnetic field detectors 6 and the like, it is sufficient to grasp relative positions to the subject 1 in advance. By using the relative positions, position detection and detection of the orientation is possible without disposing the magnetic field detectors 6 in a cube shape. The number of the magnetic field detectors 6 and the like is not limited to eight. As the simplest configuration, a system using a single magnetic field detector 6 or the like can be constructed. That is, the test capsule 2 or the capsule endoscope 25 as the body-insertable device does not travel arbitrarily in the subject 1 but travels along a path that is determined to a certain degree of predetermined organs such as esophagus, stomach, small intestine, large intestine, and the like. Therefore, it is possible to preliminarily grasp a travel path of the body-insertable device to a certain degree. The position of the body-insertable device can be detected by using the path information grasped in advance and the intensity of the constant magnetic field detected by the single magnetic field detector.

Further, in the first and second embodiments, the reference device and the selected devices are selected by using the reference device selector 42 and the selector 17, and position detection is made based on the magnetic field intensities detected by the reference device and the selected devices. The configuration, however, is not essential to the invention. For example, it is also possible to derive the distance from the test capsule 2 or capsule endoscope 25 based on detected intensities with respect to all of the magnetic field detectors 6a to 6h and generate eight ways of equations similar to Equations (2) to (5) to derive the position of the test capsule 2 or the like. In the case of the configuration, computation using, for example, the least square method is possible. Thus, there is an advantage such that an error in derivation of the position of the test capsule 2 or the like can be further reduced.

Similarly, for example in the second embodiment, the orientation of the capsule endoscope 25 may be derived by using a plurality of magnetic field detectors 6. That is, it is preferable to derive more accurate orientation by employing a method of deriving the orientations of the plurality of magnetic field detectors 6 by the method and deriving an average of the obtained orientations. The method may be similarly applied to detection of the position of the body-insertable device. A configuration of performing position detection a plurality of times by using the magnetic field detectors 6 or the like in different combinations and averaging positions obtained by the position detections may be also employed.

Although the function executing unit 32 having the CCD 30 and the like as an image capturing unit and the LED 28 and the like as an illuminating unit has been described in the second embodiment, the function executing unit may be configured to obtain information of pH and the temperature in the subject 1. A configuration that the body-insertable device has an oscillator to capture an ultrasonic image of the inside of the subject 1 may be also employed. Further, a plurality of pieces of information may be obtained from the information of the inside of the subject.

The radio signal output from the power supply antennas B1 to Bm is not always limited to a signal obtained by multiplexing the control information signal and the power supply signal. Further, radio transmission from the position transducer to the capsule endoscope may not be performed. Alternately, the power supply signal and a signal other than the control information signal may be multiplexed and the resultant signal may be transmitted. The position transducer 26 may receive only the radio signal output from the capsule endoscope. It is also possible to provide a storage unit in the capsule endoscope and, after the capsule endoscope is excreted to the outside of the subject 1, read information from the storage unit.

Although selection of the power supply antennas B1 to Bm is not mentioned in the second embodiment, like the case of the receiving antennas A1 to An, an optimum power supply antenna may be selected based on the position and orientation of the capsule endoscope 25 to perform radio transmission. To improve the efficiency of supplying the power supply signal and the like, radio signals are not unconditionally transmitted from all of power supply antennas but, by using the orientation of the capsule endoscope 25 or the like, an antenna adapted to the orientation or the like of the receiving antenna unit 36 provided in the capsule endoscope 25 can be selected.

INDUSTRIAL APPLICABILITY

As described above, the system for detecting a position in a subject according to the invention is useful for a swallowable capsule endoscope used in, for example, the medical field and, particularly, suitable for a body-insertable device such as a capsule endoscope whose position in a subject such as a patient is detected.

**Claims**

1. A system for detecting a position in a subject (1), comprising a body-insertable capsule device (2; 25) that is adapted to the introduced into the subject (1) and to travel in the subject (1), and a position transducer (3; 26) that is disposed on the outside of the subject (1) and is adapted to obtain information of a position of the body-insertable device (2; 25) in the subject (1), wherein

   the body-insertable device (2; 25) has a magnetic field generator (12) that is adapted to output a constant magnetic field to the outside of the subject (1), and

   the position transducer (3; 26) includes

   a magnetic field detector (6a to 6h) that is adapted to sense magnetic fields;

   a magnetic field extractor (15) that is adapted to eliminate a noise magnetic field component from the magnetic fields sensed by the magnetic field detector (6a to 6h) and to extract a constant magnetic field output from the magnetic field generator (12);

   a position information processor (10; 27) that is adapted to derive information of the position of the body-insertable device (2; 25) in the subject (1) based on the intensity of the constant magnetic field extracted by the magnetic field extractor (15);

   **characterized by** further comprising:

   a noise magnetic field component storage unit (14) that is adapted to store a direction and intensity of a noise magnetic field component sensed by the magnetic field detector (6a to 6h);

   a vertical direction sensor (7) that is adapted to sense a correspondence relation between a vertical direction and a coordinate system in the magnetic field detector (6a to 6h); and

   a horizontal in-plane azimuth sensor (8) that is adapted to sense a correspondence relation between an azimuth in a reference horizontal plane and a coordinate system in the magnetic field detector (6a to 6h), wherein

   the magnetic field extractor (15) is adapted to extract a constant magnetic field output from the magnetic field generator (12) by deriving a difference value between the magnetic field sensed by the magnetic field detector (6a to 6h) and the noise magnetic field component stored in the noise magnetic field component storage unit (14),

   the magnetic field extractor (15) is adapted to derive the difference value in each azimuth between the magnetic field sensed by the magnetic field detector (6a to 6h) and the noise magnetic field component stored in the noise magnetic field component storage unit (14) based on a correspondence relation sensed by the vertical direction sensor (7) and the horizontal in-plane azimuth sensor (8) to eliminate the noise magnetic field component, and

   the noise magnetic field component storage unit (14) is adapted to store a noise magnetic field component including at least an earth magnetic component existing in a region in which the capsule (2) is introduced into the subject (1) as the noise magnetic field component.

2. The system for detecting a position in a subject (1) according to claim 1, wherein

   the magnetic field detector (6a to 6h) is disposed in a state where it is fixed to the subject (1), and

   the vertical direction sensor (7) has an inclination sensor that is adapted to sense inclination of the subject (1) from the vertical direction.

**3.** The system for detecting a position in a subject (1) according to claim 1, wherein
the position transducer (3; 26) has a plurality of magnetic field detectors (6a to 6h), and
the position information processor (10; 27) includes

a distance calculator (18) that is adapted to derive a distance between the body-insertable device and each of the plurality of magnetic field detectors (6a to 6h) based on the intensity of a constant magnetic field extracted by the magnetic field extractor (15) from the results of detection of the plurality of magnetic field detectors (6a to 6h); and
a position calculator (19) that is adapted to derive the position of the body-insertable device (2; 25) by performing a predetermined computing process based on the derived distance.

**4.** The system for detecting a position in a subject (1) according to claim 1, wherein
the magnetic field generator (12) is disposed in a state where the magnetic field output direction is fixed to the body-insertable device (25), and
the position transducer (26) further includes an orientation direction detector (45) that is adapted to detect orientation of the body-insertable device (25) in the subject (1) based on the travel direction of the constant magnetic field extracted by the magnetic field extractor (15).

**5.** The system for detecting a position in a subject (1) according to claim 4, wherein
the position transducer (26) further includes an orientation direction database (44) in which correspondence relations among distance from the magnetic field generator (12), the direction of the magnetic field component extracted by the magnetic field extractor (15), and the orientation of the body-insertable device (25) are preliminarily recorded, and
the orientation direction detector (45) is adapted to detect the orientation of the body-insertable device (25) based on the correspondence relations stored in the orientation direction database (44).

**6.** The system for detecting a position in a subject (1) according to claim 1, wherein
the body-insertable device (25) further includes

a predetermined function executing unit (32) that is adapted to obtain information of the inside of the subject (1); and
a radio transmitting unit (33) that is adapted to transmit the information of the inside of the subject (1) obtained by the function executing unit (32) by radio, and

the position transducer (26) further includes a receiving unit (A1 to An) that is adapted to receive a radio signal transmitted from the radio transmitting unit (33).

**7.** The system for detecting a position in a subject (1) according to claim 6, wherein
a plurality of receiving units (A1 to An) are disposed, and
the position transducer (26) further includes a selector that is adapted to select the receiving unit (A1 to An) used for receiving a radio signal based on the position and orientation of the body-insertable device (25), derived by the position information processor (27).

**8.** The system for detecting a position in a subject (1) according to claim 6, wherein
the function executing unit (32) includes

an illuminating unit (28) that is adapted to illuminate the inside of the subject; and
an image capturing unit (30) that is adapted to capture an image of a region illuminated by the illuminating unit (28).

**9.** The system for detecting a position in a subject (1) according to claim8 wherein the position transducer (26) further includes a storage unit (49) that is adapted to store an image captured by the image capturing unit (30) and the position of the body-insertable device (25) at the time of capturing the image so that the image and the position are associated with each other.

**Patentansprüche**

**1.** System zum Detektieren einer Position in einem Subjekt (1), aufweisend eine in den Körper einführbare Einrichtung (2; 25), die eingerichtet ist, in das Subjekt (1) eingeführt zu werden und in dem Subjekt (1) zu wandern, und einen Positionstransducer (3; 26), der auf der Außenseite des Subjektes (1) angeordnet ist und eingerichtet ist, Informationen über eine Position der in den Körper einführbaren Einrichtung (2; 25) in dem Subjekt (1) zu gewinnen, wobei die in den Körper einführbare Einrichtung (2; 25) einen Magnetfeldgenerator (12) aufweist, der eingerichtet ist, ein konstantes Magnetfeld außenseitig des Subjektes (1) zu erzeugen, und wobei der Positionstransducer (3; 26) folgendes aufweist:

einen Magnetfelddetektor (6a bis 6h), der eingerichtet ist, Magnetfelder zu detektieren;
einen Magnetfeldextraktor (15), der eingerichtet ist, eine Rauschkomponente des Magnetfeldes aus dem vom Magnetfelddetektor (6a bis 6h) detektierten Magnetfeld zu eliminieren und der eingerichtet ist, ein konstantes Magnetfeld zu extrahieren, welches vom Magnetfeldgenerator (12) erzeugt ist;
einen Positionsinformationsprozessor (10; 27), der eingerichtet ist, Informationen bezüglich der Position der in den Körper einführbaren Einrichtung (2; 25) in dem Subjekt (1) auf Basis der Intensität des konstanten Magnetfeldes abzuleiten, welches von dem Magnetfeldextraktor (15) extrahiert worden ist;

**dadurch gekennzeichnet, dass** es weiterhin folgendes aufweist:

eine Speichereinheit (14) für die Rauschkomponente des Magnetfeldes, welche eingerichtet ist, eine Richtung und Intensität der Rauschkomponente des Magnetfeldes, wie vom Magnetfelddetektor (6a bis 6h) detektiert, zu speichern;
einen Vertikalrichtungssensor (7), der eingerichtet ist, eine funktionale Beziehung zwischen einer vertikalen Richtung und einem Koordinatensystem in dem Magnetfelddetektor (6a bis 6h) zu ermitteln; und
einen horizontalen Azimutsensor (8) in der Ebene, der eingerichtet ist, eine funktionale Beziehung zwischen einem Azimut in einer horizontalen Bezugsebene und einem Koordinatensystem im Magnetfelddetektor (6a bis 6h) zu ermitteln, wobei

der Magnetfeldextraktor (15) eingerichtet ist, ein konstantes Magnetfeld zu extrahieren, welches vom Magnetfeldgenerator (12) erzeugt wird durch Ableitung eines Differenzwertes zwischen dem vom Magnetfelddetektor (6a bis 6h) detektierten Magnetfeld und der Rauschkomponente des Magnetfeldes, die in der Speichereinheit (14) für die magnetische Rauschkomponente abgespeichert ist,
der Magnetfeldextraktor (15) eingerichtet ist, den Differenzwert für jeden Azimut zwischen dem vom Magnetfelddetektor (6a bis 6h) detektieren Magnetfeld und der Rauschkomponente des Magnetfeldes in der Speichereinrichtung für die Magnetfeld-Rauschkomponente abzuleiten auf Basis der funktionalen Beziehung, wie von dem Vertikalrichtungssensor (7) und dem horizontalten Azimutsensor (8) in der Ebene detektiert worden ist, um die Rauschkomponente des Magnetfeldes zu eliminieren, und wobei
die Speichereinheit (14) für die Rauschkomponente des Magnetfeldes eingerichtet ist, eine Magnetfeld-Rauschkomponente einschließlich zumindest der Erdfeldmagnetkomponente im Bereich der Kapsel (2), welche in das Subjekt (1) eingeführt ist, als Rauschkomponente des Magnetfeldes zu speichern.

2. System zum Detektieren einer Position in einem Subjekt (1) gemäß Anspruch 1, bei dem
der Magnetfelddetektor (6a bis 6h) in einem Zustand positioniert wird, in dem er am Subjekt (1) befestigt ist, und
der Vertikalrichtungssensor (7) einen Neigungssensor hat, der eingerichtet ist, eine Neigung des Subjektes (1) in Bezug auf die Vertikalrichtung zu detektieren.

3. System zum Detektieren einer Position in einem Subjekt (1) gemäß Anspruch 1, bei dem
der Positionstransducer (3; 26) eine Mehrzahl von Magnetfelddetektoren (6a bis 6h) aufweist, und
der Positionsinformationsprozessor (10; 27) einen Distanzrechner (18) enthält, der eingerichtet ist, einen Abstand zwischen der in den Körper einführbaren Einrichtung und jedem der mehreren Magnetfelddetektoren (6a bis 6h) aus den Ergebnissen der Detektion der mehreren Magnetfelddetektoren (6a bis 6h) abzuleiten auf Basis der Intensität eines konstanten, vom Magnetfeldextraktor (15) extrahierten Magnetfeldes; und
einen Positionsrechner (19), der eingerichtet ist, die Position der in den Körper einführbaren Einrichtung (2; 25) durch Rechnung auf Basis des abgeleiteten Abstandes zu ermitteln.

4. System zum Detektieren einer Position in einem Subjekt (1) gemäß Anspruch 1, bei dem
der Magnetfeldgenerator (12) in einem Zustand positioniert wird, in dem die Ausgaberichtung des Magnetfeldes in Bezug auf die in den Körper einführbare Einrichtung (25) fixiert ist und
der Positionstransducer (26) weiterhin einen Orientierungsrichtungsdetektor (45) aufweist, der eingerichtet ist, die Orientierung der in den Körper einführbaren Einrichtung (25) in dem Subjekt (1) auf Basis der Bewegungsrichtung des konstanten Magnetfeldes zu ermitteln, welches durch den Magnetfeldextraktor (15) extrahiert ist.

**5.** System zum Detektieren einer Position in einem Subjekt (1) gemäß Anspruch 4, bei dem
der Positionstransducer (26) weiterhin eine Orientierungsrichtungsdatenbasis (44) aufweist, in welcher funktionale Beziehungen zwischen einem Abstand vom Magnetfeldgenerator (12), die Richtung der vom Magnetfeldextraktor (15) extrahierten Magnetfeldkomponente, und die Orientierung der in den Körper einführbaren Einrichtung (25) vorläufig abgespeichert werden, und
der Orientierungsrichtungsdetektor (45) eingerichtet ist, die Orientierung der in den Körper einführbaren Einrichtung (25) auf Basis der funktionalen Beziehungen, die in der Orientierungsrichtungsdatenbasis (44) abgespeichert sind, zu detektieren.

**6.** System zum Detektieren einer Position in einem Subjekt (1) gemäß Anspruch 1, bei dem
die in den Körper einführbare Einrichtung (25) weiterhin aufweist:

eine Ausführungseinheit (32) für eine vorgegebene Funktion, welche eingerichtet ist, Informationen bezüglich des Inneren des Subjektes (1) zu gewinnen; und
eine Radioübertragungseinheit (33), die eingerichtet ist, die Information bezüglich des Inneren des Subjektes (1), welche durch die Funktionsausführungseinheit (32) gewonnen wurde, durch Radiowellen zu übertragen, und wobei
der Positionstransducer (26) weiterhin eine Empfangseinheit (A1 bis An) aufweist, die eingerichtet ist, die von der Radionübertragungseinheit (33) übertragenen Radiosignale zu empfangen.

**7.** System zum Detektieren einer Position in einem Subjekt (1) gemäß Anspruch 6, bei dem
eine Mehrzahl von Empfangseinheiten (A1 bis An) angeordnet sind und
der Positionstransducer (26) weiterhin einen Selektor aufweist, der eingerichtet ist, die Empfangseinheit (A1 bis An) auszuwählen, welche verwendet wird zum Empfang eines Radiosignals, und zwar auf Basis der Position und Orientierung der in den Körper einführbaren Einrichtung (25), welche vom Positionsinformationsprozessor (27) abgeleitet ist.

**8.** System zum Detektieren einer Position in einem Subjekt (1) gemäß Anspruch 6, bei dem
die Funktionsausführungseinheit (32) folgendes aufweist:

eine Beleuchtungseinheit (28), welche eingerichtet ist, das Innere des Subjektes zu beleuchten; und
eine Bildaufnahmeeinheit (30), die eingerichtet ist, ein Bild eines Bereichs aufzunehmen, welcher durch die Beleuchtungseinheit (28) beleuchtet ist.

**9.** System zum Detektieren einer Position eines Subjektes (1) gemäß Anspruch 8, bei dem der Positionstransducer (26) weiterhin eine Speichereinheit (49) aufweist, welche eingerichtet ist, ein mit der Bildaufnahmeeinheit (30) gewonnenes Bild und die Position der in den Körper einführbaren Einrichtung (25) zur Zeit der Bildaufnahme zu speichern, so dass das Bild und die Position einander zugeordnet sind.

## Revendications

**1.** Système pour détecter une position dans un sujet (1), comprenant un dispositif (2 ; 25) de type capsule pouvant être inséré dans un corps qui est adapté pour être introduit dans le sujet (1) et pour se déplacer dans le sujet (1), et un transducteur de position (3 ; 26) qui est disposé à l'extérieur du sujet (1) et est adapté pour obtenir des informations d'une position du dispositif (2 ; 25) pouvant être inséré dans un corps dans le sujet (1), dans lequel
le dispositif (2 ; 25) pouvant être inséré dans un corps comporte un générateur (12) de champ magnétique qui est adapté pour délivrer en sortie un champ magnétique constant vers l'extérieur du sujet (1), et
le transducteur de position (3 ; 26) comprend

un détecteur (6a à 6h) de champ magnétique qui est adapté pour détecter des champs magnétiques ;
un extracteur (15) de champ magnétique qui est adapté pour éliminer une composante de bruit de champ magnétique des champs magnétiques détectés par le détecteur (6a à 6h) de champ magnétique et pour extraire un champ magnétique constant délivré en sortie depuis le générateur (12) de champ magnétique ;
un processeur (10 ; 27) d'informations de position qui est adapté pour dériver des informations de la position du dispositif (2 ; 25) pouvant être inséré dans un corps dans le sujet (1) sur la base de l'intensité du champ magnétique constant extrait par l'extracteur (15) de champ magnétique ;

**caractérisé en ce qu'**il comprend en outre :

une unité (14) de mémorisation de composante de bruit de champ magnétique qui est adaptée pour mémoriser une direction et une intensité d'une composante de bruit de champ magnétique détecté par le détecteur (6a à 6h) de champ magnétique ;

un capteur (7) de direction verticale qui est adapté pour détecter une relation de correspondance entre une direction verticale et un système de coordonnées dans le détecteur (6a à 6h) de champ magnétique ; et

un capteur (8) d'azimut dans le plan horizontal qui est adapté pour détecter une relation de correspondance entre un azimut dans un plan horizontal de référence et un système de coordonnées dans le détecteur (6a à 6h) de champ magnétique, dans lequel

l'extracteur (15) de champ magnétique est adapté pour extraire un champ magnétique constant délivré en sortie depuis le générateur (12) de champ magnétique en dérivant une valeur de différence entre le champ magnétique détecté par le détecteur (6a à 6h) de champ magnétique et la composante de bruit de champ magnétique mémorisée dans l'unité (14) de mémorisation de composante de bruit de champ magnétique,

l'extracteur (15) de champ magnétique est adapté pour dériver la valeur de différence dans chaque azimut entre le champ magnétique détecté par le détecteur (6a à 6h) de champ magnétique et la composante de bruit de champ magnétique mémorisée dans l'unité (14) de mémorisation de composante de bruit de champ magnétique sur la base d'une relation de correspondance détectée par le capteur (7) de direction verticale et le capteur (8) d'azimut dans le plan horizontal pour éliminer la composante de bruit de champ magnétique, et

l'unité (14) de mémorisation de composante de bruit de champ magnétique est adaptée pour mémoriser une composante de bruit de champ magnétique incluant au moins une composante magnétique terrestre se trouvant dans une région dans laquelle la capsule (2) est introduite dans le sujet (1) en tant que composante de bruit de champ magnétique.

2. Système de détection d'une position dans un sujet (1) selon la revendication 1, dans lequel
le détecteur (6a à 6h) de champ magnétique est disposé dans un état dans lequel il est fixé au sujet (1), et
le capteur (7) de direction verticale comporte un capteur d'inclinaison qui est adapté pour détecter une inclinaison du sujet (1) par rapport à la direction verticale.

3. Système de détection d'une position dans un sujet (1) selon la revendication 1, dans lequel
le transducteur de position (3 ; 26) comporte une pluralité de détecteurs (6a à 6h) de champ magnétique, et
le processeur (10 ; 27) d'informations de position comprend

un calculateur de distance (18) qui est adapté pour dériver une distance entre le dispositif pouvant être inséré dans un corps et chacun parmi la pluralité de détecteurs (6a à 6h) de champ magnétique sur la base de l'intensité d'un champ magnétique constant extrait par l'extracteur (15) de champ magnétique à partir des résultats de détection de la pluralité de détecteurs (6a à 6h) de champ magnétique ; et

un calculateur de position (19) qui est adapté pour dériver la position du dispositif (2 ; 25) pouvant être inséré dans un corps en exécutant un traitement de calcul prédéterminé sur la base de la distance dérivée.

4. Système de détection d'une position dans un sujet (1) selon la revendication 1, dans lequel
le générateur (12) de champ magnétique est disposé dans un état dans lequel la direction de sortie de champ magnétique est fixée au dispositif (25) pouvant être inséré dans un corps, et
le transducteur de position (26) comprend en outre un détecteur (45) de direction d'orientation qui est adapté pour détecter l'orientation du dispositif (25) pouvant être inséré dans un corps dans le sujet (1) sur la base de la direction de déplacement du champ magnétique constant extrait par l'extracteur (15) de champ magnétique.

5. Système de détection d'une position dans un sujet (1) selon la revendication 4, dans lequel
le transducteur de position (26) comprend en outre une base de données (44) de direction d'orientation dans laquelle des relations de correspondance parmi la distance à partir du générateur (12) de champ magnétique, la direction de la composante de champ magnétique extraite par l'extracteur (15) de champ magnétique, et l'orientation du dispositif (25) pouvant être inséré dans un corps sont enregistrées au préalable, et
le détecteur (45) de direction d'orientation est adapté pour détecter l'orientation du dispositif (25) pouvant être inséré dans un corps sur la base des relations de correspondance mémorisées dans la base de données (44) de direction d'orientation.

6. Système de détection d'une position dans un sujet (1) selon la revendication 1, dans lequel

le dispositif (25) pouvant être inséré dans un corps comprend en outre

une unité (32) d'exécution de fonction prédéterminée qui est adaptée pour obtenir des informations de l'intérieur du sujet (1) ; et
une unité (33) de transmission radio qui est adaptée pour transmettre les informations de l'intérieur du sujet (1) obtenues par l'unité (32) d'exécution de fonction par radio, et

le transducteur de position (26) comprend en outre une unité de réception (A1 à An) qui est adaptée pour recevoir un signal radio transmis depuis l'unité (33) de transmission radio.

7. Système de détection d'une position dans un sujet (1) selon la revendication 6, dans lequel
une pluralité d'unités de réception (A1 à An) sont disposées, et
le transducteur de position (26) comprend en outre un sélecteur qui est adapté pour sélectionner l'unité de réception (A1 à An) utilisée pour recevoir un signal radio sur la base de la position et de l'orientation du dispositif (25) pouvant être inséré dans un corps, dérivées par le processeur (27) d'informations de position.

8. Système de détection d'une position dans un sujet (1) selon la revendication 6, dans lequel
l'unité (32) d'exécution de fonction comprend

une unité d'éclairage (28) qui est adaptée pour éclairer l'intérieur du sujet ; et
une unité (30) de capture d'image qui est adaptée pour capturer une image d'une région éclairée par l'unité d'éclairage (28).

9. Système de détection d'une position dans un sujet (1) selon la revendication 8, dans lequel le transducteur de position (26) comprend en outre une unité de mémorisation (49) qui est adaptée pour mémoriser une image capturée par l'unité (30) de capture d'image et la position du dispositif (25) pouvant être inséré dans un corps au moment de la capture de l'image de sorte que l'image et la position sont associées l'une à l'autre.

# FIG.1

SUBJECT
1

TEST CAPSULE
(BODY-INSERTABLE DEVICE)
2

POSITION TRANSDUCER
3

FIXING MEMBER
9a

MAGNETIC FIELD DETECTOR
6a

INCLINATION SENSOR
7

HORIZONTAL IN-PLANE
AZIMUTH SENSOR
8

POSITION INFORMATION
PROCESSOR
10

DISPLAY
4

PORTABLE RECORDING MEDIUM
5

EP 1 698 265 B1

# FIG.2

CASING
11

S    N

13
FILLING MEMBER

12
PERMANENT MAGNET
(MAGNETIC FIELD GENERATOR)

# FIG.3

EP 1 698 265 B1

# FIG.4

```
        ┌──────────┐
        │  START   │
        └──────────┘
              │
              ▼
┌─────────────────────────────┐
│  EXTRACT CONSTANT MAGNETIC   │    S101
│  FIELD OUTPUT FROM PERMANENT │
│  MAGNET                      │
└─────────────────────────────┘
              │
              ▼
┌─────────────────────────────┐
│  SELECT MAGNETIC FIELD       │    S102
│  DETECTOR AS SELECTED DEVICE │
│  BASED ON REFERENCE DEVICE   │
└─────────────────────────────┘
              │
              ▼
┌─────────────────────────────┐
│  SELECT MAGNETIC FIELD       │    S103
│  DETECTOR AS SELECTED DEVICE │
│  BASED ON REFERENCE DEVICE   │
└─────────────────────────────┘
              │
              ▼
┌─────────────────────────────┐
│  OBTAIN MAGNETIC FIELD       │    S104
│  INTENSITIES RECEIVED BY     │
│  REFERENCE DEVICE AND        │
│  SELECTED DEVICE             │
└─────────────────────────────┘
              │
              ▼
┌─────────────────────────────┐
│      CALCULATE DISTANCE      │    S105
└─────────────────────────────┘
              │
              ▼
┌─────────────────────────────┐
│  CALCULATE POSITION OF TEST  │    S106
│  CAPSULE BASED ON DISTANCE   │
└─────────────────────────────┘
              │
              ▼
┌─────────────────────────────┐
│ RECORD POSITION OF TEST      │    S107
│ CAPSULE                      │
└─────────────────────────────┘
              │
              ▼
        ┌──────────┐
        │   END    │
        └──────────┘
```

# FIG.5

# FIG.6

PERMANENT MAGNET
12

CASE MEMBER
21

LIQUID
22

SPHERICAL BODY
23

WEIGHTING MEMBER
24

11
CASING

13
FILLING MEMBER

S

N

EP 1 698 265 B1

# FIG.7

SUBJECT
1

25
CAPSULE ENDOSCOPE
(BODY-INSERTABLE DEVICE)

A4    A5
6d    6c
6b

INCLINATION SENSOR
7

FIXING MEMBER
9a

MAGNETIC FIELD DETECTOR
6a

RECEIVING ANTENNA
A1

HORIZONTAL IN-PLANE
AZIMUTH SENSOR
8

POSITION INFORMATION
PROCESSOR
27

POSITION TRANSDUCER
26

A6    6h    6g
A2    A3

An

6e    9b    6f

EP 1 698 265 B1

# FIG.8

**FUNCTION EXECUTING UNIT**
*32*

**CAPSULE ENDOSCOPE** *25*

*28*
**LED (ILLUMINATING UNIT)**

*29*
**LED DRIVING CIRCUIT**

**TRANSMITTING ANTENNA UNIT** *34*

*30*
**CCD (IMAGE CAPTURING UNIT)**

*33*
**RF TRANSMITTING UNIT (RADIO UNIT)**

**PERMANENT MAGNET** *12*

| N | S |
|---|---|

*31*
**CCD DRIVING CIRCUIT**

*35*
**SYSTEM CONTROL CIRCUIT**

*41*
**CONTROL INFORMATION DETECTING CIRCUIT**

*37*
**SEPARATING CIRCUIT**

*38*
**POWER REPRODUCING CIRCUIT**

*39*
**BOOSTER CIRCUIT**

*40*
**CAPACITOR**

*36*
**RECEIVING ANTENNA UNIT**

28

EP 1 698 265 B1

FIG.9

POSITION INFORMATION PROCESSOR 27

STORAGE UNIT 49
IMAGE PROCESSING UNIT 48
RF RECEIVING UNIT 47
ANTENNA SELECTOR 46
CONTROL INFORMATION INPUT UNIT 51
OSCILLATOR 50
ORIENTATION DIRECTION DB 44
POSITION CALCULATOR 19
MULTIPLEXING CIRCUIT 52
ORIENTATION DIRECTION DETECTOR 45
DISTANCE CALCULATOR 18
POWER SUPPLY UNIT 54
AMPLIFIER CIRCUIT 53
REFERENCE DEVICE SELECTOR 16
SELECTOR 17
MAGNETIC FIELD EXTRACTOR 15
NOISE MAGNETIC FIELD COMPONENT DB 14

RECEIVING ANTENNA A1
RECEIVING ANTENNA A2
RECEIVING ANTENNA An
POWER SUPPLY ANTENNA B1
POWER SUPPLY ANTENNA Bm
MAGNETIC FIELD DETECTOR 6a 6b 6h
INCLINATION SENSOR 7
HORIZONTAL IN-PLANE AZIMUTH SENSOR 8

29

# FIG.10

START

RECEIVE POSITION OF CAPSULE
ENDOSCOPE AND MAGNETIC FIELD
DIRECTION RECEIVED BY SELECTED
MAGNETIC FIELD DETECTOR — S201

DERIVE POSITION OF SELECTED DEVICE
RELATIVE TO CAPSULE ENDOSCOPE — S202

ENTER RELATIVE POSITION AND
MAGNETIC FIELD DIRECTION TO
ORIENTATION DIRECTION DATABASE
AND DERIVE ORIENTATION DIRECTION — S203

OUTPUT ORIENTATION TO ANTENNA
SELECTOR AND STORAGE UNIT — S204

END

# FIG.11

MAGNETIC FIELD
DETECTOR
6

$(a_1, a_2, a_3)$

$(x_1, y_1, z_1)$

CAPSULE
ENDOSCOPE
25

$(x, y, z)$

**EP 1 698 265 B1**

**Patent documents cited in the description**

- JP 2003019111 A
- WO 027281 A
- US 20030130792 A